# EUROPÄISCHE PATENTANMELDUNG

(11) **EP 3 858 306 A1**
(43) Veröffentlichungstag der Anmeldung: **04.08.2021**
(21) Anmeldenummer: 20154862.5
(22) Anmeldetag: 31.01.2020
(51) Int. Cl.: A61F 9/06, A61F 9/02

(54) **SCHWEISSHELM**

(71) Anmelder: FRONIUS INTERNATIONAL GmbH, 4643 Pettenbach (AT)
(72) Erfinder: BEZRUCKA, Patrick, 4643 Pettenbach (AT); FRIEDL, Helmut, 4643 Pettenbach (AT)
(74) Vertreter: Sonn & Partner Patentanwälte

(57) **Zusammenfassung**

Die Erfindung betrifft einen Schweißhelm (1) mit zumindest einer Helmschale (2), Befestigungselementen (3), einer Blendschutzeinrichtung (4) und zumindest einer davon beabstandeten Schutzscheibe (5) und zumindest einer Anzeigeeinrichtung (6) zur Darstellung von Daten oder dgl. im Blickfeld eines Schweißers. Zur Erzielung einer besonders einfachen und kostengünstigen Ausführungsvariante eines Schweißhelms 1 ist die zumindest eine Anzeigeeinrichtung (6) im Randbereich des Blickfelds im Zwischenraum (8) zwischen Blendschutzeinrichtung (4) und Schutzscheibe (5) angeordnet, dadurch wird ein Schutz der zumindest einen Anzeigeeinrichtung (6) vor Staub und Schmutz sowie mechanischen Belastungen erreicht.

## Beschreibung

Die Erfindung betrifft einen Schweißhelm mit zumindest einer Helmschale, Befestigungselementen, einer Blendschutzeinrichtung und zumindest einer davon beabstandeten Schutzscheibe und zumindest einer Anzeigeeinrichtung zur Darstellung von Daten oder dgl. im Blickfeld eines Schweißers.

Seit Langem ist es üblich die Augen des Schweißers gegen das grelle Licht des beim Schweißen auftretenden Lichtbogens zu schützen. Ursprüngliche Visiere werden mehr und mehr durch Schweißhelme mit einer Blendschutzeinrichtung ersetzt, die neben der Schutzfunktion für die Augen auch eine mechanische Schutzfunktion für den Kopf des Schweißers sowie einen Schutz vor dem Einatmen giftiger Dämpfe, die beim Schweißen auftreten können, bieten. Die Blendschutzeinrichtung kann verschiedenartig ausgebildet sein, beispielsweise in Form einer beide Augen des Schweißers schützenden Einrichtung oder zwei getrennte Einrichtungen für jedes Auge des Schweißers. Während früher einfache abgedunkelte Folien zum Einsatz kamen, werden heutzutage automatisch abdunkelnde Einrichtungen in Form sogenannter Blendschutzkassetten verwendet.

Neben dem Abdunkeln der Blendschutzeinrichtung, z.B. nach dem Zünden des Lichtbogens oder durch eine entsprechende Fernsteuerung, werden auch zunehmend verschiedene Daten oder dgl. im Blickfeld des Schweißers, der den Schweißhelm trägt, eingeblendet. Dadurch muss der Schweißer während der Durchführung des Schweißprozesses die Augen nicht von der Schweißstelle abwenden, um Daten (z.B. Schweißparameter) oder dgl. von einer Anzeige des Schweißgeräts oder dgl. abzulesen. Unter den Begriff Schutzscheibe fallen üblicherweise Vorsatzscheiben, welche vor der Blenschutzeinrichtungg angeordnet sind. Bei manchen Ausführungen von Schweißhelmen lässt sich die Blendschutzeinrichtung aber auch entfernen oder in Art eines Visiers hochklappen und eine dahinter im Schweißhelm angeordnete Schutzscheibe schützt die Augen des Schweißers. Die Schutzscheibe kann auch als optisches Glas ausgeführt sein.

Beispielsweise beschreibt die EP 3 192 481 A1 eine Schweißmaske bzw. einen Schweißhelm mit einer Blendschutzeinrichtung sowie einem "Head-Up-Display" zum Einblenden von Schweißparametern oder dgl.

Die EP 2 488 135 B1 beschreibt einen Schweißhelm mit integrierter Benutzerschnittstelle, wobei die Darstellung von Daten oder dgl. so erfolgt, dass diese in derselben Fokusebene wie der Schweißprozess selbst angezeigt werden. Dadurch muss sich der Schweißer vom Schweißprozess nicht abwenden, um die gewünschten Daten oder dgl. ablesen zu können.

Schließlich beschreibt die US 2016/0163221 A1 einen Schweißhelm mit einer darunter angeordneten Datenbrille, in die Schweißparameter in das Blickfeld des Schweißers eingeblendet werden können, sodass dieser die Daten auch ohne Abwenden des Blicks von der Schweißstelle ablesen kann.

Abgesehen davon, dass in vielen Fällen die Daten oder dgl. das Blickfeld des Schweißers zu sehr einschränken und einen ungehinderten Blick auf die Schweißnaht verhindern, sind die Anzeigeeinrichtungen bekannter Schweißhelme oft ungeschützt bzw. unzureichend geschützt angeordnet, sodass diese Staub, Schweißrauch und sonstigen Verschmutzungen sowie mechanischen Belastungen ausgesetzt sind. Ein weiterer Nachteil besteht darin, dass bisherige Anzeigen in Schweißhelmen oder Datenbrillen allfällige Fehlsichtigkeiten des Schweißers nicht berücksichtigen.

Die Aufgabe der vorliegenden Erfindung besteht in der Schaffung eines oben genannten Schweißhelms, mit zumindest einer Anzeigeeinrichtung zur Darstellung von Daten oder dgl., welche Anzeigeeinrichtung vor den beim Schweißen üblichen Umwelteinflüssen geschützt ist, sodass eine möglichst lange Lebensdauer gewährleistet ist. Das Blickfeld des Schweißers sollte durch die dargestellten Daten oder dgl. möglichst wenig eingeschränkt werden, um einen möglichst freien Blick auf die Schweißnaht gewährleisten zu können. Der Schweißhelm soll weiters möglichst einfach und kostengünstig aufgebaut sein, um eine breite Anwendung sicherstellen zu können. Nachteile bekannter Schweißhelme sollen verhindert oder zumindest reduziert werden.

Gelöst wird die erfindungsgemäße Aufgabe dadurch, dass die zumindest eine Anzeigeeinrichtung im Randbereich des Blickfelds des Schweißers im Zwischenraum zwischen der Blendschutzeinrichtung und zumindest einer Schutzscheibe angeordnet ist. Erfindungsgemäß wird die zumindest eine Anzeigeeinrichtung in besonders einfacher Weise in einem vorhandenen Zwischenraum zwischen Blendschutzeinrichtung des Schweißhelms und Schutzscheibe angeordnet, wodurch die Anzeigeeinrichtung vor Staub, Schweiß, Rauch, sonstigen Verschmutzungen sowie mechanischen Belastungen und anderen Umwelteinflüssen optimal geschützt werden kann. Dabei werden zur Vermeidung eines Schwindelgefühls ("Motion Sickness") des Schweißers beispielsweise die Daten oder dgl. nur bei Bedarf oder nach entsprechender Aufforderung und nicht permanent eingeblendet. Wenn zwei Anzeigeeinrichtungen rechts und links des Blickfelds des Schweißers im Schweißhelm angeordnet werden, können für das linke und rechte Auge des Schweißers unterschiedliche Daten oder dgl. eingeblendet werden oder es kann durch das Einblenden derselben Information ein dreidimensionaler Effekt hervorgerufen werden. Dadurch, dass die zumindest eine Anzeigeeinrichtung im Randbereich des Blickfelds des Schweißers im Schweißhelm angeordnet ist, wird der Blick auf den Arbeitsbereich in sehr geringem Ausmaß eingeschränkt, wobei der Schweißer durch entsprechende Augenbewegung rasch die auf der Anzeigeeinrichtung dargestellten Daten oder dgl. ablesen kann ohne den Blick von der Schweißung abzuwenden. Heutige Technik erlaubt eine miniaturisierte Realisierung der zumindest einen Anzeigeeinrichtung wodurch eine Anordnung auch bei geringen Zwischenräumen zwischen Blendschutzeinrichtung und Schutzscheibe möglich wird. Darüber hinaus sind derartige Anzeigeeinrichtungen zu sehr geringen Kosten erhältlich, wodurch ein Nachrüsten eines Schweißhelms mit zumindest einer solchen Einrichtung ebenfalls denkbar ist. Auch kann ein Umrüsten des Schweißhelms auf verschiedene Anzeigeeinrichtungen rasch und einfach vorgenommen werden. Durch das Einblenden der Daten oder dgl. über den Schweißprozess während der Durchführung des Schweißprozesses braucht der Schweißer den Blick vom Arbeitsbereich nicht abwenden, wodurch eine optimale Schweißqualität erzielt werden kann. Durch die geschützte Anordnung der zumindest einen Anzeigeeinrichtung im Zwischenraum kann der Schweißhelm besonders lange ohne Wartung der Anzeigeeinrichtung eingesetzt werden.

Wenn die zumindest eine Anzeigeeinrichtung verstellbar im Zwischenraum angeordnet ist, kann rasch und einfach eine Anpassung der Position der Anzeigeeinrichtung in Bezug auf das Auge bzw. die Augen des Schweißers erzielt werden. Die Verstellbarkeit der Anzeigeeinrichtung kann durch verschiedene mechanische Konstruktionen, wie z.B. Stellschrauben oder dgl., aber auch elektrische Konstruktionen, wie z.B. Schrittmotoren oder dgl., erfolgen. Bei der Anordnung zweier Anzeigeeinrichtungen im Schweißhelm, kann durch die Verstellbarkeit auch eine Anpassung an die Position der Augen des Schweißers zueinander vorgenommen werden.

Dabei ist es besonders vorteilhaft, wenn die zumindest eine Anzeigeeinrichtung über zumindest einen Antrieb verstellbar angeordnet ist. Durch zumindest einen solchen vorzugsweise elektrischen Antrieb, kann die Verstellung der zumindest einen Anzeigeeinrichtung auch während des Tragens des Schweißhelms vorgenommen werden, bis der Schweißer ein optimales Resultat erhält. Die Verstellung des zumindest einen Antriebs kann auch mit Hilfsmitteln, wie z.B. Smartphones, Notebooks oder dgl. über entsprechende Apps oder Softwareprogramme in besonders bequemer Art und Weise vorgenommen werden. Die Einstellungen können vorzugsweise auch gespeichert werden, sodass diese bei Bedarf wieder abgerufen und auf den jeweiligen Schweißer, der den Schweißhelm trägt, angepasst werden können.

Zwischen Blendschutzeinrichtung und der zumindest einen Anzeigeeinrichtung kann eine Dichtung, vorzugsweise aus elastischem Material, angeordnet sein. Durch eine solche, vorzugsweise elastische, Dichtung wird die zumindest eine Anzeigeeinrichtung vor Vibrationen besser geschützt und auch ein Eindringen von Schmutz in das Innere der Anzeigeeinrichtung verhindert werden. Ein Austauschen der Anzeigeeinrichtung wird durch eine solche Dichtung, welche mit der Blendschutzeinrichtung vorzugsweise nicht fix verbunden ist, erleichtert, da nach Abnahme der Schutzscheibe die Anzeigeeinrichtung bloß ausgewechselt und die Dichtung zwischen Anzeigeeinrichtung und Blendschutzeinrichtung platziert wird. Die Dichtung kann auch fest mit dem Gehäuse der Anzeigeeinrichtung verbunden sein, wodurch die Anzeigeeinrichtung automatisch bei der Anordnung im Zwischenraum zwischen Blendschutzeinrichtung und Schutzscheibe gegenüber der Blendschutzeinrichtung abgedichtet wird.

Vorteilhafterweise ist die zumindest eine Anzeigeeinrichtung mit einer vorhandenen Steuereinrichtung verbunden. Die Verbindung zwischen Anzeigeeinrichtung und Steuereinrichtung des Schweißhelms kann drahtgebunden vorzugsweise über geeignete Steckverbindungen oder auch drahtlos erfolgen. Über die Verbindung werden sowohl Daten als auch die Versorgungsspannung zu den jeweiligen Komponenten übertragen. Dadurch wird eine Nachrüstung des Schweißhelms mit zumindest einer Anzeigeeinrichtung oder ein Tausch derselben in einfacher Weise möglich. Die Steuereinrichtung kann sowohl im Schweißhelm selbst integriert oder angeordnet sein, oder auch in einem davon beabstandeten Gerät. Über die Steuereinrichtung des Schweißhelms kann die Anzeigeeinrichtung optimal angesteuert werden und somit definiert werden, welche Daten oder dgl. an der Anzeigeeinrichtung dargestellt und dem Schweißer bei Bedarf zur Verfügung gestellt werden. Die Steuereinrichtung ist üblicherweise durch einen Mikroprozessor gebildet, der zur Erfüllung entsprechender Steuerungen der Blendschutzeinrichtung und der zumindest einen Anzeigeeinrichtung des Schweißhelms entsprechend programmiert wird.

Die Blendschutzeinrichtung kann im Bereich der zumindest einen Anzeigeeinrichtung zumindest eine Ausnehmung aufweisen. In diesem Fall ist keine Blendschutzeinrichtung angeordnet wo die zumindest eine Anzeigeeinrichtung angeordnet ist, wodurch der Blick zur Anzeigeeinrichtung durch die Blendschutzeinrichtung nicht eingeschränkt wird. Ebenso kann an Stellen wo eine Anzeigeeinrichtung angeordnet ist auf die unter Umständen aufwändige und teure Blendschutzeinrichtung verzichtet werden. Alternativ dazu kann die Blendschutzeinrichtung auch an den Stellen der Anzeigeeinrichtung angeordnet sein, wobei an diesen Stellen jedoch keine Abdunkelung während des Schweißvorgangs vorgenommen wird, sodass der Blick auf die Anzeigeeinrichtung nicht beeinträchtigt wird.

Wenn die zumindest eine Anzeigeeinrichtung mit der Schutzscheibe verbunden ist, kann ein einfacher und rascher Tausch der Anzeigeeinrichtung durch Demontage und nachfolgender Montage der Schutzscheibe erfolgen. Die Verbindung zwischen Anzeigeeinrichtung und Schutzscheibe kann beispielsweise durch Kleben, Schrauben oder dgl. erfolgen.

Die zumindest eine Anzeigeeinrichtung beinhaltet vorzugsweise eine Anzeige, insbesondere eine Farbanzeige, und zumindest eine Optik. Derartige Anzeigeeinrichtungen sind in geringen Baugrößen und zu besonders niedrigen Preisen erhältlich. Die Verbindung der Anzeigeeinrichtung mit einer entsprechenden Steuereinrichtung und der erforderlichen Spannungsversorgung erfolgt üblicherweise über eine entsprechende Kabelverbindung. Allenfalls kann eine Spannungsversorgung auch in Form einer kleinen Batterie oder eines Akkumulators in der Anzeigeeinrichtung enthalten sein und die Verbindung mit einer Steuereinrichtung auch drahtlos erfolgen. Die Optik kann durch eine Linse, einen Spiegel oder einen Lichtleiter, der das anzuzeigende Bild an die gewünschte Stelle leitet, oder auch Kombinationen davon gebildet sein.

Zwischen der zumindest einen Anzeigeeinrichtung und der Blendschutzeinrichtung kann zumindest eine Korrekturoptik angeordnet sein, um Fehlsichtigkeiten und Stellungsfehler der Augen des Schweißers ausgleichen zu können. Zur individuellen Anpassung an den jeweiligen Schweißer ist es von Vorteil, wenn die zumindest eine zusätzliche Korrekturoptik einfach austauschbar ist.

Wenn die zumindest eine Anzeigeeinrichtung mit zumindest einem Sensor verbunden ist, können weitere Funktionen erzielt werden. Durch die Verbindung des jeweiligen Sensors mit einer Steuereinrichtung im Schweißhelm können verschiedene hilfreiche Funktionen erzielt werden.

Dabei kann zumindest ein Sensor an der der Schutzscheibe zugewandten Seite der Anzeigeeinrichtung angeordnet sein. Mit einem derartigen Sensor kann eine Steuerung der Anzeigeeinrichtung oder anderer Funktionen des Schweißhelms bzw. des Schweißsystems oder der Schweißanlage vorgenommen werden. Beispielsweise kann über einen mechanischen Sensor durch manuelle Betätigung im Bereich der Schutzscheibe eine Umschaltung von Funktionen des Schweißhelms bzw. des Schweißsystems durchgeführt werden. Über entsprechende optische (z.B. Infrarotsensoren), kapazitive, induktive oder andere Sensoren, kann die Steuerung auch berührungslos erfolgen indem der Schweißer durch Platzierung seiner Hand vor dem Sensor eine Umschaltung bewirkt. Aber auch andere Sensoren, wie z.B. Lichtsensoren, können zur Steuerung der Helligkeit der Anzeige der Anzeigeeinrichtung herangezogen werden.

Zumindest ein Sensor kann durch eine Kamera, ein Mikrophon, einen Lichtsensor, einen Berührungssensor und bzw. oder einen Temperatursensor gebildet sein. Bei der Verwendung einer Kamera als Sensor, kann durch Gestensteuerung mit der Hand des Schweißers eine Umschaltung der Anzeigeeinrichtung oder anderer Funktionen des Schweißhelms bzw. des Schweißsystems vorgenommen werden. Über zumindest ein Mikrophon kann beispielsweise der Umgebungslärm bzw. die beim Schweißen durch den Lichtbogen entstehenden Töne aufgezeichnet oder zur Steuerung bestimmter Funktionen verwendet werden. Denkbar ist beispielsweise auch die Steuerung der Anzeigeeinrichtung oder anderer Funktionen des Schweißhelms bzw. des Schweißsystems über Sprache. Bewegungssensoren sowie Positionssensoren oder Gyroskopsensoren können zur Steuerung in Abhängigkeit der Position oder Lage des Schweißhelms herangezogen werden.

Wenn am Schweißhelm ein Nasenausschnitt vorgesehen ist, und die zumindest eine Anzeigeeinrichtung neben dem Nasenausschnitt angeordnet, resultiert ein optimale und platzsparende Anordnung der zumindest eine Anzeigeeinrichtung, ohne dass der Blick auf den Arbeitsbereich eingeschränkt wird. Je nach Gestaltung des Schweißhelms kann auch die Anordnung der zumindest einen Anzeigeeinrichtung oberhalb des Nasenausschnitts zweckmäßig sein.

Gemäß einem weiteren Merkmal der Erfindung ist die Blendschutzeinrichtung durch eine Blendschutzkassette gebildet. Eine derartige Blendschutzkassette ist üblicherweise durch eine Flüssigkristallanzeige gebildet und kann besonders rasch zwischen verschiedenen Abdunkelungsgraden umgeschaltet werden. Dadurch kann beispielsweise bei einem kurzschlussbehafteten Schweißprozess die Blende während der Kurzschlüsse, währenddessen kein Lichtbogen brennt, aufgehellt werden, wodurch der Blick auf die Schweißnaht verbessert wird. An Stellen der Blendschutzkassette, an welchen mehr Schutz benötigt wird, kann die Verdunkelung lokal oder punktuell auch stärker eingestellt werden.

Wenn die zumindest eine Anzeigeeinrichtung mit einem Transceiver verbunden ist, kann eine Übertragung der Daten zu einer Zentrale bzw. zu einer entsprechenden Steuereinrichtung erfolgen. Die Steuereinrichtung kann am oder im Schweißhelm oder auch davon entfernt angeordnet sein. Der Transceiver kann sowohl drahtgebunden als auch drahtlos arbeiten.

Die vorliegende Erfindung wird anhand der beigefügten Zeichnungen näher erläutert. Darin zeigen:
- Fig. 1: einen üblichen Schweißhelm in seitlicher, teilweise geschnittenen Ansicht, mit zumindest einer Helmschale, Befestigungselementen, einer Blendschutzeinrichtung, zumindest einer Schutzscheibe und zumindest einer Anzeigeeinrichtung zur Darstellung von Daten oder dgl. im Blickfeld eines Schweißers;
- Fig. 2: eine seitliche, teilweise geschnittene Ansicht auf einen erfindungsgemäßen Schweißhelm mit einer im Zwischenraum zwischen Blendschutzeinrichtung und Schutzscheibe am unteren Rand des Blickfelds angeordneten Anzeigeeinrichtung;
- Fig. 3: eine seitliche, teilweise geschnittene Ansicht auf eine andere Ausführungsform eines Schweißhelms mit einer im Zwischenraum zwischen Blendschutzeinrichtung und Schutzscheibe am unteren Rand des Blickfelds angeordneten Anzeigeeinrichtung;
- Fig. 4: eine alternative Ausführungsform eines Schweißhelms mit einer im Zwischenraum zwischen Blendschutzeinrichtung und Schutzscheibe am oberen Rand des Blickfelds angeordneten Anzeigeeinrichtung;
- Fig. 5: eine weitere alternative Ausführungsform eines Schweißhelms mit einer im Zwischenraum zwischen Blendschutzeinrichtung und Schutzscheibe angeordneten Anzeigeeinrichtung;
- Fig. 6: eine Detailansicht verschiedener Ausführungsformen verstellbar angeordneter Anzeigeeinrichtungen;
- Fig. 7: eine Detailansicht einer anderer Ausführungsform einer Anzeigeeinrichtung mit Korrekturoptik;
- Fig. 8: schematisch zwei neben einem Nasenausschnitt am Schweißhelm angeordneter Anzeigeeinrichtungen;
- Fig. 9: ein geschnittenes Detail einer Anzeigeeinrichtung mit einer Kamera zur Erfassung von Gesten des Schweißers; und
- Fig. 10: eine schematische Darstellung einer mit der Steuereinrichtung und verschiedenen Sensoren verbundenen Anzeigeeinrichtung für einen Schweißhelm.

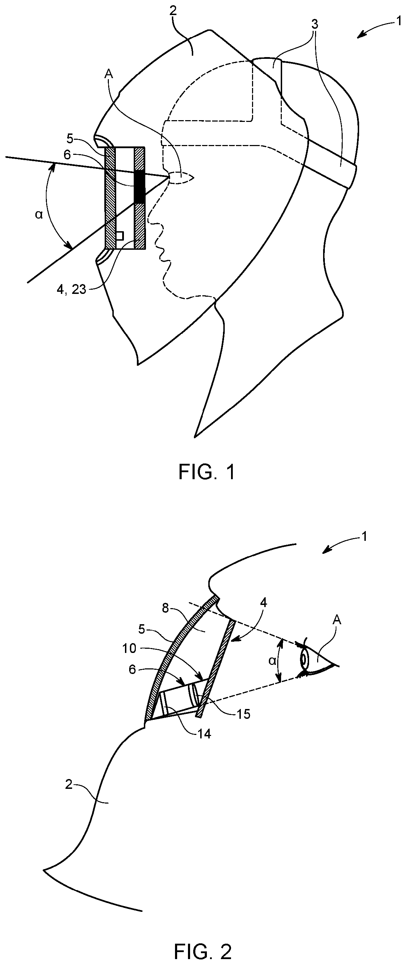

Fig. 1 zeigt einen üblichen Schweißhelm 1 in seitlicher, teilweise geschnittenen Ansicht, mit zumindest einer Helmschale 2, entsprechenden Befestigungselementen 3, wie z.B. Riemen oder Gurte, einer Blendschutzeinrichtung 4, zumindest einer Schutzscheibe 5 und zumindest einer Anzeigeeinrichtung 6 zur Darstellung von Daten oder dgl. im Blickfeld eines Schweißers. In der Darstellung ist eine Schutzscheibe 5 in Form einer Vorsatzscheibe vor der Blendschutzeinrichtung 4 angeordnet. Es sind auch Schweißhelme 1 bekannt, wo die Blenschutzeinrichtungg 4 zusammen mit der Schutzscheibe 5 in Form einer Vorsatzscheibe in Art eines Visiers hochgeklappt werden kann und dahinter eine weitere Schutzscheibe 5 zum Schutz der Augen des Schweißers bei hochgeklapptem Visier angeordnet ist (nicht dargestellt). Bei den an der Anzeigeeinrichtung 6 darzustellenden Daten oder dgl. handelt es sich üblicherweise um Schweißparameter wie z.B. Stromstärke, Drahtvorschub, Spannung, Schweißjob, aber auch Fehlermeldungen, Rückmeldungen positiver Art (z.B. Schweißnaht ok) und Warnungen, welche für den Schweißer von Interesse sind. Bei üblichen Schweißhelmen 1 befindet sich die zumindest eine Anzeigeeinrichtung 6 im Blickfeld des den Schweißhelm 1 tragenden Schweißers und ist beispielsweise durch ein entsprechendes "Head-Up-Display" gebildet. Der Blickwinkel des Auges A des Schweißers ist mit α eingezeichnet. Dadurch, dass die entsprechenden Daten oder dgl. im Blickfeld des Schweißers eingeblendet werden, braucht dieser den Blick von der Schweißnaht oder dem Arbeitsbereich während des Schweißprozesses nicht abwenden. Andererseits sind derartige Anzeigeeinrichtungen 6 relativ teuer und aufwändig und es kann unter Umständen durch die Anzeige entsprechender Daten oder dgl. der Blick auf den Schweißpunkt bzw. die Schweißnaht eingeschränkt werden. Die Blendschutzeinrichtung 4 ist vorzugsweise durch eine Blendschutzkassette 23 gebildet. Zur Vermeidung des Auftretens eines Schwindelgefühls des Schweißers ist es von Vorteil, dass die zumindest eine Anzeigeeinrichtung 6 nur bei Bedarf oder nach entsprechender Aufforderung und nicht permanent aktiviert wird. Dadurch wird auch der Blick auf den Arbeitsbereich nicht unnötig beeinträchtigt.

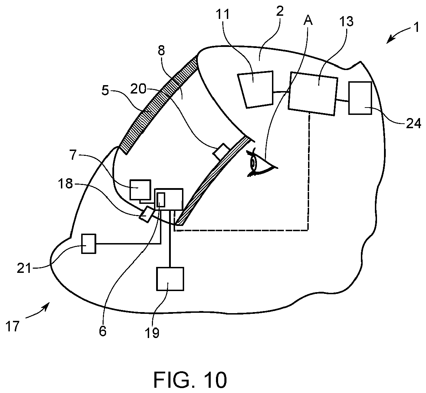

Fig. 2 zeigt eine seitliche, teilweise geschnittene Ansicht auf einen erfindungsgemäßen Schweißhelm 1 mit einer im Zwischenraum 8 zwischen Blendschutzeinrichtung 4 und Schutzscheibe 5 am unteren Rand des Blickfelds des Schweißers angeordneten Anzeigeeinrichtung 6. Dadurch, dass die zumindest eine Anzeigeeinrichtung 6 im Zwischenraum 8 zwischen Blendschutzeinrichtung 4 und Schutzscheibe 5 angeordnet ist, wird diese vor Schmutz, Staub, etc. geschützt und kann daher lange ohne Wartung eingesetzt werden. Darüber hinaus ist die zumindest eine Anzeigeeinrichtung 6 vor mechanischer Belastung optimal geschützt. Durch die Anordnung der zumindest einen Anzeigeeinrichtung 6 im Randbereich des Blickfelds des Schweißers wird der Blickwinkel α des Schweißers möglichst wenig gestört aber dennoch ein Ablesen der relevanten Daten oder dgl. von der Anzeigeeinrichtung 6 in einfacher Weise ermöglicht. Zwischen der Anzeigeeinrichtung 6 und der Blendschutzeinrichtung 4 ist vorzugsweise eine Dichtung 10 angeordnet. Diese Dichtung 10 ist vorzugsweise aus elastischem Material, insbesondere Kunststoffmaterial, gebildet. Durch die Dichtung 10 wird die Anzeigeeinrichtung 6 vor mechanischen Stößen besser geschützt und zusätzlich ein allfälliges Verschmutzen der Anzeigeeinrichtung 6 verhindert. Die Anzeigeeinrichtung 6 wird über entsprechende Kabel und Steckverbindungen mit einer Steuereinrichtung des Schweißhelms 1 verbunden (s. Fig. 10). Die Anzeigeeinrichtung 6 kann mit der Schutzscheibe 5 verbunden sein, um einen Austausch möglichst einfach und rasch vornehmen zu können. Die Anzeigeeinrichtung 6 besteht zumindest aus einer Anzeige 14 und einer entsprechenden Optik 15, wobei die Anzeige 14 vorzugsweise durch eine Farbanzeige gebildet ist. Die Optik 15 kann durch eine Linse, einen Spiegel, oder einen Lichtleiter bzw. Kombinationen davon gebildet sein.

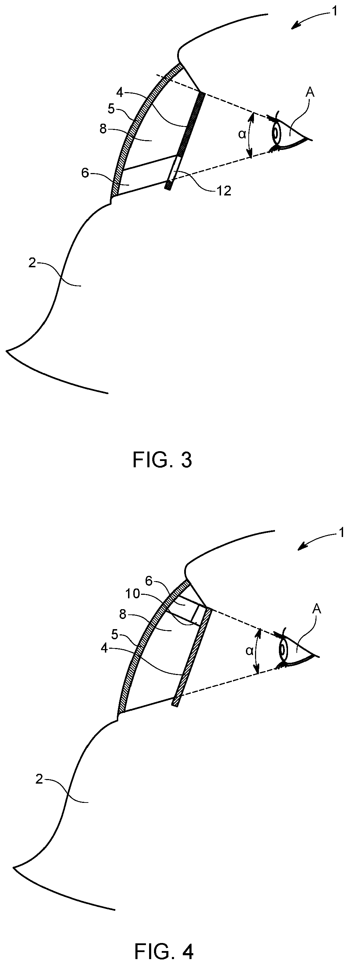

Fig. 3 zeigt eine seitliche, teilweise geschnittene Ansicht auf eine andere Ausführungsform eines Schweißhelms 1 mit einer im Zwischenraum 8 zwischen Blendschutzeinrichtung 4 und Schutzscheibe 5 am unteren Rand des Blickfelds angeordneten Anzeigeeinrichtung 6, wobei die Blendschutzeinrichtung 4 im Bereich der Anzeigeeinrichtung 6 in diesem Fall ausgenommen ist bzw. eine entsprechende Ausnehmung 12 oder ein Loch aufweist. Dadurch kann ein ungehinderter Blick des Schweißers auf die Anzeigeeinrichtung 6 auch bei ansonsten abgedunkelter Blendschutzeinrichtung 4 gewährleistet werden bzw. die, unter Umständen teure, Blendschutzeinrichtung 4 im Bereich der Anzeigeeinrichtung 6 eingespart werden.

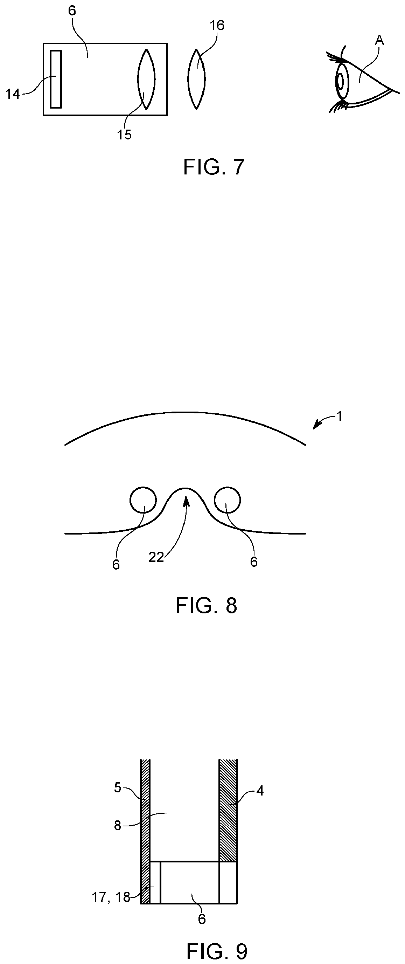

In Fig. 4 ist eine alternative Ausführungsform eines Schweißhelms 1 mit einer im Zwischenraum 8 zwischen Blendschutzeinrichtung 4 und Schutzscheibe 5 am oberen Rand des Blickfelds angeordneten Anzeigeeinrichtung 6 dargestellt. Je nach Anwendung kann es von Vorteil sein, wenn die zumindest eine Anzeigeeinrichtung 6 am oberen Rand des Blickwinkels α des Schweißers angeordnet ist. Bei Schweißhelmen 1 mit Nasenausschnitt 22 eignet sich jedoch der Bereich neben dem Nasenausschnitt 22 für die Anordnung einer oder zweier Anzeigeeinrichtungen 6 besonders (siehe Fig. 8). Bei der Anordnung zweier Anzeigeeinrichtungen 6 können unterschiedliche Daten oder dgl. für das linke und rechte Auge A des Schweißers eingeblendet werden oder durch das Anordnen gleicher grafischer Informationen ein dreidimensionaler Effekt erzielt werden.

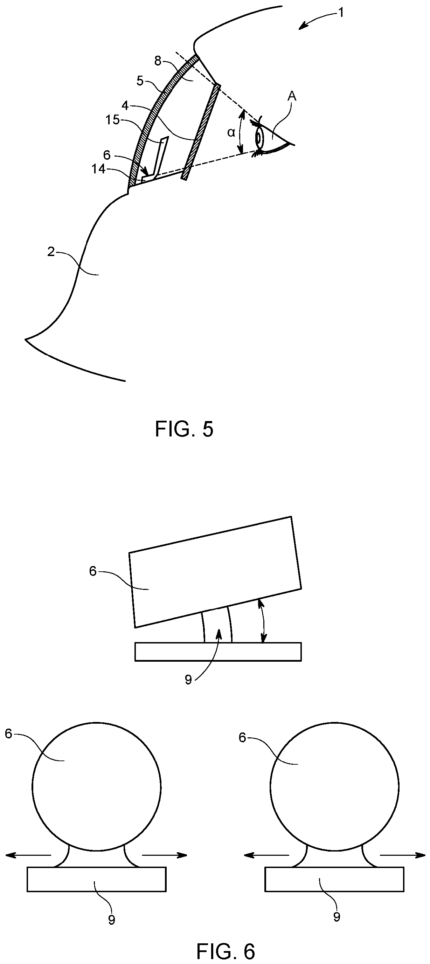

In Fig. 5 ist eine weitere alternative Ausführungsform eines Schweißhelms 1 mit einer im Zwischenraum 8 zwischen Blendschutzeinrichtung 4 und Schutzscheibe 5 angeordneten Anzeigeeinrichtung 6 dargestellt. Die Anzeigeeinrichtung 6 weist eine Anzeige 14 und eine Optik 15 in Form eines im Zwischenraum nach oben weisenden Lichtleiters auf. Der Lichtleiter als Optik 15 leitet das Bild der Anzeige 14 in Art eines Head-up-Displays weiter.

Fig. 6 zeigt eine Detailansicht verschiedener Ausführungsformen verstellbar angeordneter Anzeigeeinrichtungen 6. Zur Anpassung der zumindest einen Anzeigeeinrichtung 6 an die Stellung der Augenart des Schweißers sind vorzugsweise entsprechende Mittel zur verstellbaren Anordnung der zumindest einen Anzeigeeinrichtung 6 vorgesehen. Diese Mittel zur Verstellung können durch einfache mechanische oder aufwändigere elektromechanische Konstruktionen gebildet sein. Beispielsweise kann ein seitliches oder vertikales Verschieben der zumindest einen Anzeigeeinrichtung 6 durch entsprechende Einstellschrauben (nicht dargestellt) erzielt werden. Aber auch mit entsprechenden Antrieben 9 kann ein Kippen oder Verschieben der zumindest einen Anzeigeeinrichtung 6 erzielt werden. Durch ein seitliches Verschieben der zumindest einen Anzeigeeinrichtung 6 kann diese an den Abstand der Augen des Schweißers optimal angepasst werden und eine optimale Sicht auf die Anzeigeeinrichtung 6 gewährleistet werden. Wie dargestellt, kann für jedes Auge eine separate Anzeigeeinrichtung 6 vorgesehen sein.

Aus Fig. 7 geht eine Detailansicht einer anderen Ausführungsform einer Anzeigeeinrichtung 6 hervor. In diesem Fall ist vor der aus einer Anzeige 14 und einer Optik 15 bestehenden Anzeigeeinrichtung 6 zumindest eine zusätzliche Korrekturoptik 16 angeordnet um Fehlsichtigkeiten oder Sehfehler des Auges A des Schweißers korrigieren zu können. Die Korrekturoptik 16 kann auch in der Anzeigeeinrichtung 6 integriert oder dieser direkt vorgesetzt sein, sollte jedoch möglichst einfach und rasch wechselbar sein um eine individuelle Anpassung an den Schweißer gewährleisten zu können.

In Fig. 8 sind schematisch zwei neben einem Nasenausschnitt 22 am Schweißhelm 1 angeordnete Anzeigeeinrichtungen 6 dargestellt. Durch eine derartige Anordnung wird das Blickfeld des Schweißers durch die Anzeigeeinrichtungen 6 besonders wenig gestört und dennoch eine optimale Ablesung der Daten oder dgl. von den Anzeigeeinrichtungen 6 gewährleistet. Zusätzlich lässt sich ein Schweißhelm 1 mit einem solchen Nasenausschnitt 22 besonders einfach und kostengünstig mit Anzeigeeinrichtungen 6 nachrüsten.

Fig. 9 zeigt ein geschnittenes Detail einer im Zwischenraum 8 zwischen Blendschutzeinrichtung 4 und Schutzscheibe 5 angeordneten Anzeigeeinrichtung 6 mit einer Kamera 18 als Sensor 17 zur Erfassung von Gesten des Schweißers. Durch einen Sensor 17 an der der Schutzscheibe 5 zugewandten Seite der Anzeigeeinrichtung 6, kann eine Steuerung der Anzeigeeinrichtung 6 oder anderer Funktionen des Schweißgeräts bzw. Schweißsystems über den Sensor 17 erfolgen. Bei der Verwendung einer Kamera 18 können beispielsweise Gesten, die mit der Hand des Schweißers vorgenommen werden, zur Auslösung bestimmter Funktionen oder zur Aktivierung oder Umschaltung der Anzeigeeinrichtung 6 herangezogen werden. Ein solcher Sensor 17 bzw. eine Kamera 18 kann besonders klein und auch kostengünstig ausgebildet sein. Auch kann mit einer Kamera 18 die Schweißnaht aufgenommen und für spätere Dokumentationszwecke oder zur Qualitätssicherung verwendet werden.

Schließlich zeigt Fig. 10 eine schematische Darstellung einer mit der Steuereinrichtung 13 und verschiedenen Sensoren 17 verbundenen Anzeigeeinrichtung 6 für einen Schweißhelm 1. Die zumindest eine Anzeigeeinrichtung 6 wird über entsprechende Kabel oder auch drahtlos mit der Steuereinrichtung 13, die sich vorzugsweise ohnedies im Schweißhelm 1 befindet, verbunden. Durch entsprechende Programmierung der Steuereinrichtung 13, welche üblicherweise durch einen Mikrocontroller oder dgl. gebildet ist, kann das Einblenden der entsprechenden Daten oder dgl. in die zumindest eine Anzeigeeinrichtung 6 gesteuert werden. Durch die Verbindung der Anzeigeeinrichtung 6 bzw. Steuereinrichtung 13 mit entsprechenden Sensoren 17 können verschiedene Zusatzfunktionen erzielt werden. Sensoren 17 können durch eine Kamera 18, ein Mikrophon 19, einen Lichtsensor 20 oder auch einen Temperatursensor 7 gebildet sein. Die Spannungsversorgung der zumindest einen Anzeigeeinrichtung 6 sowie der Steuereinrichtung 13 und der allfälligen Sensoren 17 wird über eine vorzugsweise im Schweißhelm 1 angeordnete Energieversorgung 11 bewerkstelligt. Zur Energieversorgung kann auch ein Akkumulator eines bei vielen Schweißhelmen 1 angeordneten Gebläses herangezogen werden (nicht dargestellt). Wenn die Anzeigeeinrichtung 6 bzw. die Steuereinrichtung 13 mit einem Transceiver 24 verbunden ist, kann eine Übertragung und ein Empfang von Daten oder Informationen von einer übergeordneten Stelle, beispielsweise dem Schweißgerät, durchgeführt werden. Der Transceiver 24 kann sowohl drahtgebunden als auch drahtlos ausgebildet sein.

## Patentansprüche

1. Schweißhelm (1) mit zumindest einer Helmschale (2), Befestigungselementen (3), einer Blendschutzeinrichtung (4) und zumindest einer davon beabstandeten Schutzscheibe (5) und zumindest einer Anzeigeeinrichtung (6) zur Darstellung von Daten oder dgl. im Blickfeld eines Schweißers, **dadurch gekennzeichnet, dass** die zumindest eine Anzeigeeinrichtung (6) im Randbereich des Blickfelds im Zwischenraum (8) zwischen Blendschutzeinrichtung (4) und Schutzscheibe (5) angeordnet ist.

2. Schweißhelm (1) nach Anspruch 1, **dadurch gekennzeichnet, dass** die zumindest eine Anzeigeeinrichtung (6) verstellbar im Zwischenraum (8) angeordnet ist.

3. Schweißhelm (1) nach Anspruch 2, **dadurch gekennzeichnet, dass** die zumindest eine Anzeigeeinrichtung (6) über zumindest einen Antrieb (9) verstellbar angeordnet ist.

4. Schweißhelm (1) nach einem der Ansprüche 1 bis 3, **dadurch gekennzeichnet, dass** zwischen Blendschutzeinrichtung (4) und der zumindest einen Anzeigeeinrichtung (6) eine Dichtung (10), vorzugsweise aus elastischem Material, angeordnet ist.

5. Schweißhelm (1) nach einem der Ansprüche 1 bis 4, **dadurch gekennzeichnet, dass** die zumindest eine Anzeigeeinrichtung (6) mit einer vorhandenen Steuereinrichtung (13) verbunden ist.

6. Schweißhelm (1) nach einem der Ansprüche 1 bis 5, **dadurch gekennzeichnet, dass** die Blendschutzeinrichtung (4) im Bereich der zumindest einen Anzeigeeinrichtung (6) zumindest eine Ausnehmung (12) aufweist.

7. Schweißhelm (1) nach einem der Ansprüche 1 bis 6, **dadurch gekennzeichnet, dass** die zumindest eine Anzeigeeinrichtung (6) mit der Schutzscheibe (5) verbunden ist.

8. Schweißhelm (1) nach einem der Ansprüche 1 bis 7, **dadurch gekennzeichnet, dass** die zumindest eine Anzeigeeinrichtung (6) eine Anzeige (14), insbesondere eine Farbanzeige, und zumindest eine Optik (15) beinhaltet.

9. Schweißhelm (1) nach einem der Ansprüche 1 bis 8, **dadurch gekennzeichnet, dass** zwischen der zumindest einen Anzeigeeinrichtung (6) und der Blendschutzeinrichtung (4) zumindest eine Korrekturoptik (16) angeordnet ist.

10. Schweißhelm (1) nach einem der Ansprüche 1 bis 9, **dadurch gekennzeichnet, dass** die zumindest eine Anzeigeeinrichtung (6) mit zumindest einem Sensor (17) verbunden ist.

11. Schweißhelm (1) nach Anspruch 10, **dadurch gekennzeichnet, dass** zumindest ein Sensor (17) an der der Schutzscheibe (5) zugewandten Seite der Anzeigeeinrichtung (6) angeordnet ist.

12. Schweißhelm (1) nach Anspruch 11, **dadurch gekennzeichnet, dass** zumindest ein Sensor (17) durch eine Kamera (18), ein Mikrophon (19), einen Lichtsensor (20), einen Berührungssensor (21) und bzw. oder einen Temperatursensor (7) gebildet ist.

13. Schweißhelm (1) nach einem der Ansprüche 1 bis 12, **dadurch gekennzeichnet, dass** ein Nasenausschnitt (22) vorgesehen ist, und dass die zumindest eine Anzeigeeinrichtung (6) neben dem Nasenausschnitt (22) angeordnet ist.

14. Schweißhelm (1) nach einem der Ansprüche 1 bis 13, **dadurch gekennzeichnet, dass** die Blendschutzeinrichtung (4) durch eine Blendschutzkassette (23) gebildet ist.

15. Schweißhelm (1) nach einem der Ansprüche 1 bis 14, **dadurch gekennzeichnet, dass** die zumindest eine Anzeigeeinrichtung (6) mit einem Transceiver (24) verbunden ist.
